# EUROPEAN PATENT SPECIFICATION

(11) **EP 0 336 936 B1**
(45) Date of publication and mention of the grant of the patent: **09.03.1994**
(21) Application number: 88901177.1
(22) Date of filing: 03.12.1987
(51) Int. Cl.: A62B 7/00

(54) **RESUSCITATION DEVICE**
WIEDERBELEBUNGSVORRICHTUNG
DISPOSITIF DE REANIMATION

(30) Priority: 05.12.1986 US 938419
(43) Date of publication of application: 18.10.1989
(73) Proprietor: AUGERSCOPE, INC., Sylmar, CA 91342 (US)
(72) Inventor: IRWIN, Lawrence, Sylmar, CA 91342 (US); IRWIN, Frank, Sylmar, CA 91342 (US)
(74) Representative: Lewald, Dietrich, Dipl.-Ing.
(86) International application number: US8703181
(87) International publication number: WO8804187

(56) References cited:
- CA-A- 798 660
- FR-A- 1 201 930
- US-A- 15 192
- US-A- 2 887 104
- US-A- 3 057 347
- US-A- 3 957 046
- US-A- 4 619 640

## Description

The invention relates to a mouth to mouth resuscitation device of the kind as described in the opening part of Claim 1.

Mouth to mouth resuscitation is a valuaable, life saving expedient when properly accomplished. However, in the past, the procedure has not been wholly satisfactory for several reasons including the natural repugnance most people have of placing their mouth in contact with the patien's mouth knowing that the patient is likely to vomit during the procedure. In recent times, the possibility of transmission by the patient of acquired immune deficiency, oral herpes and other serious diseases has caused people to avoid giving mouth to mouth resuscitation. Finally, the difficulty of practicing the procedure properly causes people to shy away from even attempting to render assistance.

Because of problems of the character outlined in the preceding paragraph, many types of devices have been suggested to allegedly simplify the administration of mouth to mouth resuscitation and to avoid the spread of germs. However, most of these prior art devices are cheap, ineffective gadgets.

Some of the more effective prior art devices of which the present inventor is aware include those disclosed in US-A-3,957,046 issued to Harris; US-A-4,106,502 issued to Wilson and US-A-4,535,765 issued to Pavluceio et al. The device of the present invention is readily distinguishable from these prior art disclosures because of the unique valving arrangement embodiet within the device. This novel device permits air to flow toward the patient but precludes the flow of air and fluids back to the person administering the mouth to mouth resuscitation.

One of the major drawbacks of prior art devices which embody the so-called duck valve type valving mechanism such as is shown in FR-A-1.204.930, is the fact that during the air delivery cycle the duck valve, because of its basic construction and lack of controlled impedance to fluid flow toward the patient, cannot be relied upon to automatically and positively close the exhale and expectorating discharge port typically provided in the wall of the proximal portion of the device. In point of fact, a geometrical analysis of a prior art duck valve of the character in which the closed end extends across the entire diameter of the tube in which the valve is carried, at once reveals that the valve can only expand to approximately two-thirds of the circumference of the tube. Accordingly, a discharge port in the wall of the tube cannot be reliably closed by the duck valve construction. Unless the fluid discharge port of the device is positively sealed during delivery of air to the patient as is permitted only by the unique construction of the valve of the present invention, precisely regulated and controlled delivery of air to the patient cannot be achieved during resuscitation.

The Harris patent US-A-3957046 discloses all the features of the preamble of Claim 1 but a totally different structure that operates in an entirely different way from the device defined in the appendent claims.

In summary, as will be better understood from the description which follows, the mouth to mouth resuscitating device of the present invention comprises the features of Claim 1.

### Brief Description of the Drawings

Figure 1 is a generally schematic view illustrating the use of the mouth to mouth resuscitating device of the present invention;
Figure 2 is a side elevational cross-sectional view of the resuscitator of the present invention.
Figure 3 is a top cross-sectional view of the resuscitator illustrating in phantom lines the operation of the valving mechanism of the device.
Figure 4 is an enlarged perspective view illustrating the configuration of the valving mechanism in its normal closed position.
Figure 5 is a perspective view similar to Figure 4 but showing the valve in its open position permitting the flow of air therethrough toward the patient.

### Description of the Invention

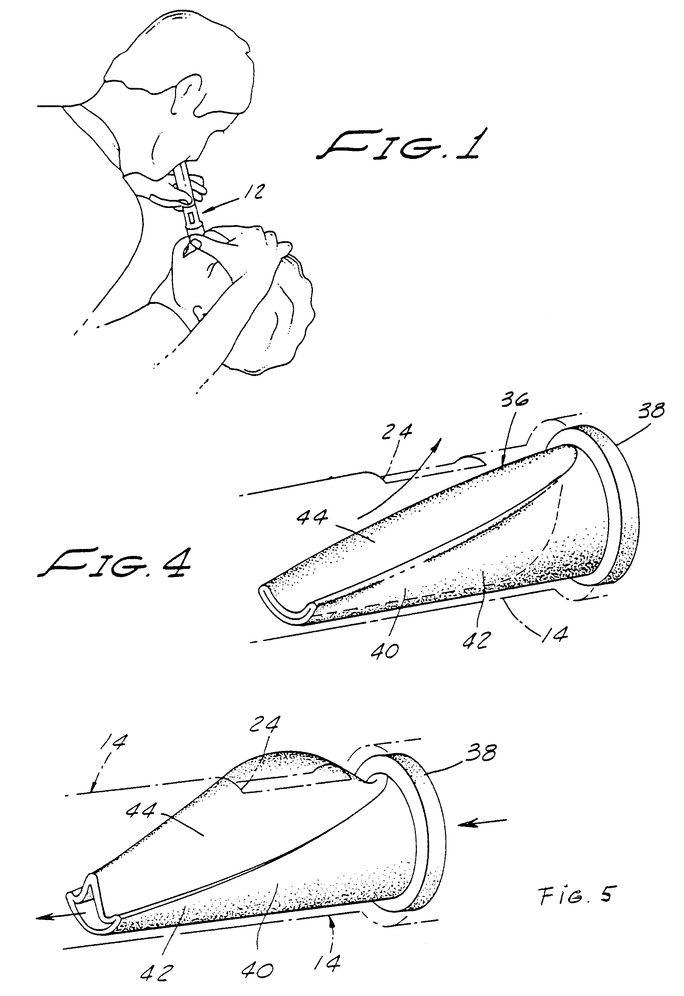

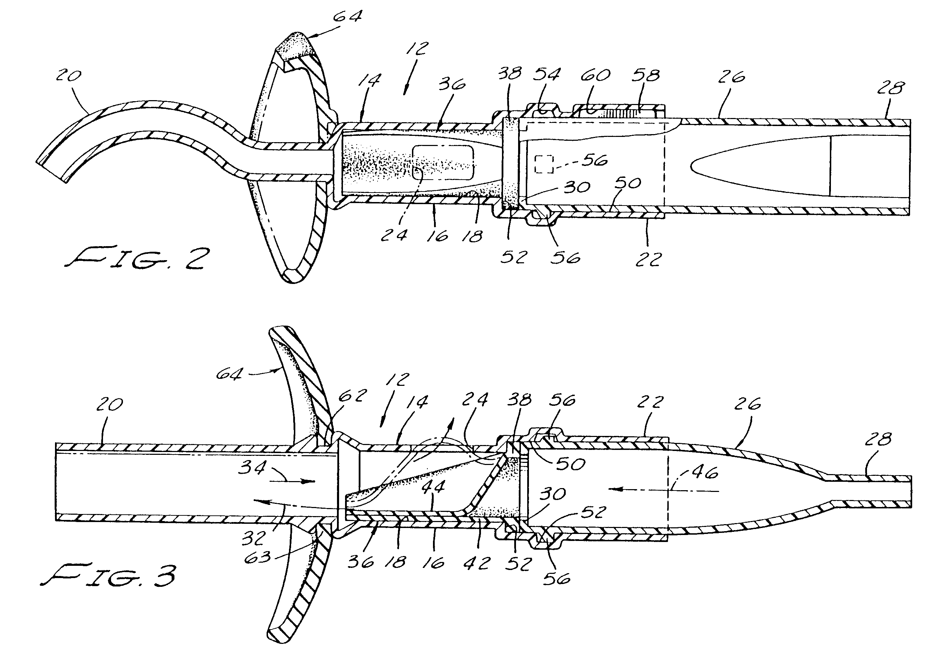

Referring to the drawings and particularly to Figures 1, 2 and 3, the mouth to mouth resuscitating device of the present invention is generally designated by the numeral 12. The device comprises a first tubular member 14 having a cylindrically shaped section 16 defining an inner longitudinally extending surface 18. First tubular member 14 has a first end portion 20 and a second end portion 22. As best seen in Figures 2 and 3, tubular portion 16 of the first member is provided with an aperture 24 extending through the wall of the tubular section.

Operably associated with first member 14 is a second tubular member 26. Member 26 has a first end portion 28 and a second end portion 30. As indicated in Figures 2 and 3, second end portion 30 of tubular member 26 is telescopically receivable within second end portion 22 of first member 14. When the first and second members are mated in the manner shown in Figures 2 and 3, they are coaxially aligned and releasably interconnected by interconnection means, the details of which will presently be described.

Forming a highly important aspect of the present invention is valving means carried within first tubular member 14 and disposed intermediate the first and second end portions thereof for permitting the flow of air through the tubular member 14 in a first direction as indicated by the arrow 32 in Figure 3, but blocking the flow of fluids through the valve in the opposite, second, direction indicated by the arrow 34.

Referring to Figures 4 and 5, in the present embodiment of the invention the valving means is provided as an integrally formed member 36 constructed of a resilient material such as rubber, neoprene, silicone or the like. Valve member 36 comprises an annular shaped, substantially rigid portion 38 and a deformable wall 40 connected to, and extending outwardly from, annular portion 38. Wall 40 has a first portion 42 which is normally disposed in close engagement with the longitudinally extending surface 18 of the first tubular member 14 (Figure 3) and a second portion 44 normally disposed in a first position in close engagement with first portion 42 of the yieldably deformable wall 40.

As illustrated in Figures 4 and 5, second portion 44 of wall 40 is yieldably deformable in response to air pressure from the first closed position shown in Figure 4 to the inflated, or outwardly extending, position shown in Figure 5. Referring to Figure 3, the solid lines illustrate the valve configuration in its normal closed position with the second portion 44 of the wall in close engagement with the first portion 42 of the wall. However, upon the flow of air through the device in the direction of the arrow designated by the numeral 46, the valve will move to its open position with second wall portion 44 moving into the orientation shown by the phantom lines in Figure 3. It is important to note that in this expanded or ballooned position, second wall 44 moves into closing engagement with the edges of aperture 24 thereby sealing the aperture and preventing any flow of fluids therethrough in the direction of the arrow generally designated by the numeral 48. The purpose of this important aperture sealing feature will be discussed in the section which follows entitled, "Operation."

As best seen by referring to Figure 3, first tubular member 14 is provided with an enlarged diameter portion 50 which is adapted to closely receive annular shaped portion 38 of the valving member. With portion 38 of the valving member in position within enlarged diameter portion 50 and in engagement with a shoulder 52 formed at the juncture of the enlarged diameter portion and portion 16 of the first valving member, second end portion 30 of second member 26 can be moved into clamping engagement with the valving member thereby sealably securing the valving means within the assemblage formed by the first and second valve members.

Also provided within first tubular member 14 is a circumferential, internal groove 54. Closely receivable within groove 54 are circumferentially spaced protuberances 56, which protuberances form a part of the interconnection means of the embodiment of the invention shown in the drawings. Protuberances 56 are of a size such that they will pass through the first end portion 22 of the first member as the walls are yieldably outwardly deformed. In this way second member 26 can be telescopically received within the first end of the first member and mateably inserted until the protuberances snap into the circumferential groove 54. Once disposed within the groove, the protuberances will function to prevent accidental separation of the parts. As indicated in Figure 3, the side walls of the circumferential groove 54 are slightly tapered so that a separating force exerted on the first and second tubular members will cause a deformation of the first end portion 22 of the first member sufficient to permit the protuberances 56 to be moved free of the circumferential groove thereby enabling separation of the first and second tubular members.

Forming another part of the interconnection means of the present invention is an alignment means for aligning the first and second tubular members in a predetermined rotational orientation. In the embodiment of the invention shown in the drawings, this alignment means comprises a longitudinally extending protuberance 58 formed on second member 26 intermediate its ends. Longitudinally extending protuberance 58 is closely receivable within a longitudinally extending internal channel 60 formed in first member 14 proximate circumferential groove 54. Upon mating the first and second tubular members so that protuberances 56 will snap into circumferentially extending groove 54, a relative rotational movement between the first and second tubular members will cause protuberance 58 to snap into longitudinally extending channel, or cavity, 60 thereby locking the components against further accidental rotation with the parts thus rotationally aligned the end portions 20 and 28 of the first and second members will be correctly operationally aligned.

As best seen in Figure 3, first member 14 is provided with an external circumferentially extending groove 62 which closely receives a central flange 63 formed on a breathing mask 64. Breathing mask 64 functions to close off the entire mouth of the victim to compel the air being breathed into the mouth to flow into the lungs and not escape at the sides of the mouth which must be open to receive portion 20. Breathing mask 64 is preferably made of a yieldably soft plastic or rubber material which will snap into circumferentially extending groove 62 and will tend to closely conform to the patient's face during use.

### Operation

Using the device of the present invention for emergency resuscitation, the victim's mouth is opened and the curved end 20 of tubular member 14 is placed on his tongue and then slid inwardly until the breathing mask 64 engages the victim's face. The person performing the resuscitation then places the thumb and finger of one hand about the victim's nose to close off the nostrils and blows into the mouth piece 28. The air pressure flowing in the direction of the arrow 46 of Figure 3 will cause the valving means to move into the open position shown by the phantom lines in Figure 3 to permit air to flow into the first tubular member and then into the patient through curved end 20. As indicated in Figure 3, deformation of the valving member also sealably closes the aperture 24 thereby preventing leakage of air through the aperture. This ensures that all of the air being breathed into the device by the person performing the resuscitation reaches the lungs of the patient. Between breaths the nostrils are released to permit the victim to exhale. When the victim exhales, or when the person performing the resuscitation ceases to force air into the device, the valving means will automatically move into its closing position shown by the solid lines in Figure 3. In this position, the valve will prevent any flow of fluid or air in the reverse direction, that is in a direction toward the person performing the resuscitation. Air from the victim, or any fluids emitted from the patient, such as vomit, will pass through the aperture 24 and never reach the person providing the emergency assistance.

As earlier pointed out, the materials and manufacturing costs of the device of the invention are very low thereby permitting the device to be discarded after each use. However, if it is desired to reuse the device, the device can easily be disassembled and the component parts thereof sterilized as by boiling. Similarly, due to the ease of disassembly of the device, it is a simple matter to replace the valving mechanism at any time with a new valving mechansim.

Having now described the invention in detail in accordance with the requirements of the patent statutes, those skilled in this art will have no difficulty in making changes and modifications in the individual parts or their relative assembly in order to meet specific requirements or conditions. Such changes and modifications may be made without departing from the scope of the invention, as set forth in the following claims.

## Claims

1. A mouth-to-mouth resuscitating device comprising:
(a) a first tubular member (14) having a wall defining an inner, longitudinally extending surface (18) and first (20) and second (22) end portions, said wall having an aperture (24) therethrough;
(b) a second tubular member (26) having first (28) and second (30) end portions, said second member being operably interconnected and substantially coaxially aligned with said first member (14);
(c) interconnection means (54, 56) for interconnecting said first (14) and second (26) tubular members proximate the second ends (22, 30) thereof; and
(d) valving means (36) for permitting the flow of fluids through said first tubular member (14) in a first direction (open position), but blocking the flow of fluids through the valve in the opposite second direction (closed position), said valving means (36) comprising an integrally formed member including a yieldable deformable wall (40);
characterized in that said valving means (36) comprises:
(i) a rigid, annular-shaped portion (38) received between said second end portions of said first (14) and second (26) tubular members;
(ii) a yieldably deformable wall (40) connected to and extending outwardly from said annular portion (38), said wall (40) having a first convex portion (42) normally disposed in close engagement with said longitudinally extending surface (18) of said first tubular member (14) and a second concave portion (44) normally disposed in a first concave position in close engagement with the concave inner surface of said first convex portion (42) of said yieldably deformable wall (40), said second portion (44) being yieldably deformable away from said concave inner surface of said first convex portion (42) to a second convex position in response to fluid pressure to block said aperture (24) and to permit the flow of fluids through said first member (14) in said first direction (open position),
wherein, in said second position, a section of said second portion (44) of said yieldably deformable wall (40) of said valving means (36) extends through the aperture (24) in said wall of said first tubular member (14).

2. A device as defined in Claim 1, in which said annular portion (38) of said valving means (36) is sealably clamped between said second end portions (22, 30) of said first and second tubular members (14, 26) when said first and second members are releasably interconnected by said interconnection means (54, 56).

3. A device as defined in Claim 1, in which said first end portion (20, 28) of each of said first and second tubular members (14, 26) is tapered for insertion into the mouths of persons using the device.

4. A device as defined in Claim 3, in which said first end portion (20) of said first tubular member (14) is generally arcuate in shape.

## Patentansprüche

1. Mund-zu-Mund-Wiederbelebungsvorrichtung mit
(a) einem ersten röhrenförmigen Teil (14), das eine Wandung, die eine sich längs erstreckende Innenoberfläche (18) bestimmt und die eine Öffnung (24) aufweist, und erste (20) und zweite (22) Endabschnitte aufweist,
(b) einem zweiten röhrenförmigen Teil (26), das erste (28) und zweite (30) Endabschnitte aufweist und mit dem ersten Teil (14) funktional verbunden und im wesentlichen koaxial zu diesem ausgerichtet ist,
(c) einer Verbindungseinrichtung (54, 56) zum Verbinden des ersten (14) und des zweiten (26) röhrenförmigen Teils unmittelbar an deren zweiten Enden (22, 30), und
(d) einer Ventileinrichtung (36), die einen Strömungsmittelfluß durch das erste röhrenförmige Teil (14) in einer ersten Richtung (Auf-Stellung) erlaubt, aber einen Strömungsmittelfluß durch das Ventil in einer zweiten, der entgegengesetzten Richtung (Zu-Stellung) verhindert, und die ein einheitlich ausgebildetes Teil mit einer elastisch verformbaren Wandung (40) aufweist,
dadurch gekennzeichnet, daß die Ventileinrichtung (36)
(i) einen zwischen den zweiten Endabschnitten des ersten (14) und des zweiten (26) röhrenförmigen Teils aufgenommenen, starren, ringförmig geformten Abschnitt (38),
(ii) eine mit dem ringförmigen Abschnitt (38) verbundene und sich davon nach außen erstreckende, elastisch verformbare Wandung (40), die einen ersten konvexen Abschnitt (42), der sich normalerweise in engem Kontakt zu der sich längs erstreckenden Oberfläche (18) des ersten röhrenförmigen Teils (14) befindet, und einen zweiten konkaven Abschnitt (44) aufweist, der sich normalerweise in einer ersten konkaven Position in engem Kontakt zu der konkaven Innenoberfläche des ersten konvexen Abschnitts (42) der elastisch verformbaren Wandung (40) befindet, wobei der zweite Abschnitt (44) zum Blockieren der Öffnung (24) und zum Verhindern eines Strömungsmittelflusses durch das erste Teil (14) in der ersten Richtung (Auf-Stellung) mittels Strömungsmitteldruck von der konkaven Innenoberfläche des ersten konvexen Abschnitts (42) weg zu einer zweiten konvexen Position elastisch verformbar ist, und
wobei in der zweiten Stellung ein Teil des zweiten Abschnitts (44) der elastisch verformbaren Wandung (40) der Ventileinrichtung (36) sich durch die Öffnung (24) in der Wandung des ersten röhrenförmigen Teils (14) erstreckt.

2. Vorrichtung nach Anspruch 1, bei der der ringförmige Abschnitt (38) der Ventileinrichtung (36) dicht zwischen den Endabschnitten (22, 30) der ersten und zweiten röhrenförmigen Teile (14, 26) einrastet, wenn das erste und das zweite Teil mittels der Verbindungseinrichtung (54, 56) lösbar miteinander verbunden sind.

3. Vorrichtung nach Anspruch 1, bei der der jeweilige erste Endabschnitt (20, 28) des ersten und des zweiten röhrenförmigen Teils (14, 26) zur Einfuhr in die Münder von die Vorrichtung benutzenden Personen verjüngt ist.

4. Vorrichtung nach Anspruch 3, bei der der erste Endabschnitt (20) des ersten röhrenförmigen Teils (14) bogenförmig ausgebildet ist.

## Revendications

1. Dispositif de réanimation bouche à bouche comprenant:
(a) un premier élément tubulaire (14) ayant une paroi qui définit une surface intérieure orientée longitudinalement (18) et une première (20) et une seconde (22) parties d'extrémité, ladite paroi étant traversée par une ouverture (24) ;
(b) un second élément tubulaire (26) ayant une première (28) et une seconde (30) parties d'extrémité, ledit second élément étant fonctionnellement relié audit premier élément (14) et sensiblement coaxial avec ce dernier ;
(c) un moyen de liaison (54, 56) pour relier entre eux lesdits premier (14) et second (26) éléments tubulaires à proximité de leurs secondes extrémités (22, 30) ; et
(d) un moyen de vanne (36) pour permettre l'écoulement de fluides à travers ledit premier élément tubulaire (14) dans une première direction (en position ouverte), mais pour bloquer l'écoulement des fluides à travers la vanne dans la seconde direction opposée (position fermée), ledit moyen de vanne (36) comprenant un élément formé d'une seule pièce qui comporte une paroi déformable souple (40) ;
caractérisé en ce que ledit moyen de vanne (36) comprend :
(i) une partie rigide en forme d'anneau (38) reçue entre lesdites secondes parties d'extrémité dudit premier (14) et dudit second (26) éléments tubulaires ;
(ii) une paroi déformable souple (40) reliée à et s'étendant vers l'extérieur à partir de ladite partie annulaire (38), ladite paroi (40) présentant une première portion convexe (42) normalement placée en prise étroite avec ladite surface orientée longitudinalement (18) dudit premier élément tubulaire (14) et une seconde portion concave (44) normalement placée dans une première position concave en prise étroite avec la surface interne concave de ladite première portion convexe (42) de ladite paroi déformable souple (40), ladite seconde portion (44) étant déformable souple en s'éloignant de ladite surface intérieure concave de ladite première portion convexe (42) en direction d'une seconde position convexe sous l'effet de la pression des fluides pour fermer ladite ouverture (24) et pour permettre l'écoulement des fluides à travers ledit premier élément (14) dans ladite première direction (position ouverte),
dans lequel dans ladite seconde position, une partie de ladite seconde portion (44) de ladite paroi déformable souple (40) dudit moyen de vanne (36) traverse l'ouverture (24) de ladite paroi dudit premier élément tubulaire (14).

2. Dispositif selon la revendication 1, dans lequel ladite portion annulaire (38) dudit moyen de vanne (36) est immobilisée de manière étanche entre lesdites secondes parties d'extrémité (22, 30) desdits premier et second éléments tubulaires (14, 26) lorsque lesdits premier et second éléments sont reliés entre eux par ledit moyen de liaison (54, 56).

3. Dispositif selon la revendication 1, dans lequel ladite première partie d'extrémité (20, 28) de chacun desdits premier et second éléments tubulaires (14, 26) est effilée pour être introduite dans la bouche des personnes utilisant le dispositif.

4. Dispositif selon la revendication 3, dans lequel ladite première partie d'extrémité (20) dudit premier élément tubulaire (14) a une forme généralement cintrée.
